# FASCICULE DE BREVET EUROPEEN

(11) **EP 0 995 423 B1**
(45) Date de publication et mention de la délivrance du brevet: **05.09.2001**
(21) Numéro de dépôt: 99402395.0
(22) Date de dépôt: 30.09.1999
(51) Int. Cl.: A61K 7/02

(54) **Poudre cosmétique et son utilisation pour le démaquillage ou le nettoyage de la peau et des muqueuses**
Kosmetisches Pulver und dessen Verwendung als Abschminkmittel oder zur Haut und Schleimhaut Reinigung
Cosmetic powder and its use as make-up remover or for skin and mucosa cleansing

(30) Priorité: 23.10.1998 FR 9813326
(43) Date de publication de la demande: 26.04.2000
(73) Titulaire: L'OREAL, 75008 Paris (FR)
(72) Inventeur: Marion, Christine, 92330 Sceaux (FR)
(74) Mandataire: Rasson, Catherine

(56) Documents cités:
- EP-A- 0 783 883
- WO-A-96/22073
- DATABASE WPI Week 9517 Derwent Publications Ltd., London, GB; AN 95-128207 XP002107112 & JP 07 053324 A (NISSHIN OIL MILLS LTD.), 28 février 1995 (1995-02-28)

## Description

L'invention se rapporte à une poudre cosmétique, à son procédé de fabrication et à son utilisation pour le nettoyage et/ou le démaquillage de la peau, des muqueuses et/ou du cuir chevelu et notamment pour le démaquillage des compositions de maquillage sans transfert et/ou longue tenue.

Les compositions de rouges à lèvres et de fonds de teint comprennent généralement des corps gras tels que des cires et des huiles ainsi qu'une phase particulaire généralement composée de charges et de pigments. Ces compositions, lorsqu'elles sont appliquées sur la peau ou les lèvres, présentent l'inconvénient de transférer, c'est-à-dire de se déposer au moins en partie, en laissant une trace sur certains supports avec lesquels elles peuvent être mises en contact, et notamment un verre, une tasse, un vêtement ou la peau. Il s'ensuit une persistance médiocre du film sur la peau ou sur les lèvres, nécessitant de renouveler régulièrement l'application de la composition de fond de teint ou de rouge à lèvres. Par ailleurs, l'apparition de traces inacceptables sur certains vêtements et notamment sur les cols de chemisier peut empêcher certaines femmes d'utiliser ce type de maquillage.

De façon récente, est apparue une nouvelle génération de produits de maquillage dits "sans transfert" et/ou "longue tenue". Ces nouveaux produits se distinguent de ceux déjà connus par la présence d'huiles volatiles, à la place des huiles plus lourdes habituellement utilisées. En particulier, les compositions de rouge à lèvres et les fonds de teint sans transfert comprennent des huiles de silicone qui n'étaient pas utilisées dans les compositions de l'art antérieur.

Les huiles volatiles ayant tendance à s'évaporer rapidement, ces nouvelles compositions de rouges à lèvres et de fonds de teint ont la particularité de former un film de corps gras solide lorsqu'on les applique respectivement sur la muqueuse labiale ou sur la peau.

Contrairement aux compositions de maquillage de l'art antérieur qui se démaquillent facilement, les compositions de maquillage longue tenue et/ou sans transfert sont difficiles à démaquiller et les produits de démaquillage habituellement utilisés ne sont pas efficaces dans ce cas-là.

En effet, les produits moussants à base de tensioactifs moussants ou de savon sont inefficaces pour le démaquillage des produits sans transfert et ils présentent, en outre, l'inconvénient d'être décapants et donc inutilisables pour les yeux et les muqueuses. Par ailleurs, les lotions ou les gels aqueux bien que moins irritants sont tout aussi inefficaces pour le démaquillage de produits sans transfert. Quant aux émulsions ou aux compositions biphasées contenant de l'huile, elles ont une efficacité insuffisante et présentent de plus l'inconvénient de contenir des conservateurs et des tensioactifs, agents potentiels d'irritation pour les peaux et yeux sensibles.

Il existe aussi des compositions démaquillantes ne comportant que de l'huile sous forme fluide ou gélifiée. Ces compositions permettent en général de démaquiller les maquillages les plus résistants mais elles présentent l'inconvénient d'être grasses et difficiles à rincer, et de manquer de fraîcheur à l'application.

L'utilisation des huiles démaquillantes en combinaison avec des polyholosides dans des compositions de nettoyage ou démaquillage sous forme de lotions, de laits, de gels, de crémes ou de mousses est décrite dans EP-A-783883.

Il subsiste donc le besoin de disposer d'une composition permettant un démaquillage satisfaisant notamment pour le démaquillage des compositions de maquillage sans transfert.

Or, la demanderesse a constaté avec étonnement que les poudres à base d'amidon modifié et d'huile démaquillante permettaient de démaquiller parfaitement et avec une grande facilité tout maquillage y compris les maquillages longue tenue et/ou sans transfert, dans des conditions de confort, en particulier de fraîcheur, très satisfaisantes.

Certes, il est connu dans le domaine cosmétique d'utiliser des poudres de nettoyage. Toutefois, la plupart de ces poudres sont à base de tensioactifs détergents et présentent les mêmes inconvénients que les produits moussants cités plus haut. D'autres poudres présentent l'inconvénient de contenir des protéines d'origine animale ou végétale, qui constituent le support de la poudre ; or, on évite d'utiliser dans des produits cosmétiques des protéines d'origine animale. En outre, ces protéines se conservent mal au cours du temps, et certaines protéines d'origine végétale, telles que les protéines de soja, peuvent se révéler irritantes, notamment lorsqu'elles sont utilisées en une quantité importante. Par ailleurs, ces poudres sont hygroscopiques, ce qui est un inconvénient lorsque l'on souhaite utiliser la poudre telle quelle, puisqu'elle a tendance à s'humidifier et présente de ce fait une mauvaise conservation, en particulier dans les pays tropicaux humides.

La poudre selon l'invention n'a pas les inconvénients notamment d'hygroscopie ou d'irritation, des poudres de nettoyage de l'art antérieur.

La présente invention a donc pour objet une poudre cosmétique de démaquillage et/ou nettoyage, caractérisée en ce qu'elle comprend, par rapport au poids total de la composition, de 5 à 50 % en poids d'au moins un amidon modifié et de 50 à 95 % en poids d'une phase huileuse comprenant au moins une huile démaquillante choisie parmi les esters d'acide gras comportant au moins 12 atomes de carbone, le rapport pondéral phase huileuse/amidon étant égal ou supérieur à 1, et la quantité d'huile(s) démaquillante(s) étant d'au moins 10% en poids par rapport au poids total de la poudre On entend par "poudre" une substance solide divisée en particules ou grains très fins et homogènes.

La poudre selon l'invention est de préférence exempte de protéine.

Dans la poudre selon l'invention, la phase huileuse est fixée dans l'amidon modifié hydrophobe.

Cette poudre présente notamment les avantages d'être très confortable, d'être applicable sur tout type de peaux sans laisser d'effet gras malgré la quantité importante d'huile, d'être facile et rapide à utiliser, de conserver les propriétés hydratantes des corps gras et de ne pas nécessiter obligatoirement l'ajout d'un liquide tel que l'eau puisqu'elle peut être utilisée telle quelle. En outre, on peut facilement en prélever la quantité souhaitée.

En outre, du fait que cette poudre peut être obtenue sans émulsionnant et qu'elle se conserve bien, on peut éviter l'ajout d'émulsionnants et/ou de conservateurs, et ainsi obtenir une poudre beaucoup moins irritante que les produits de nettoyage classiques.

La poudre selon l'invention présente une granulométrie (ou dimension moyenne en nombre de particules) pouvant aller notamment d'environ 0,1 à 100 µm, de préférence de 0,5 à 50 µm et mieux de 1 à 10 µm. La granulométrie est mesurée avec l'appareil : "MICROTRAC X100 & SRA 150" de la société LEEDS-NORTHRUP.

L'amidon modifié utilisé dans la composition de l'invention peut être modifié par une ou plusieurs des réactions suivantes : prégélatinisation, oxydation, réticulation, estérification.

De manière plus particulière, ces réactions peuvent être réalisées de la façon suivante :
- prégélatinisation en faisant éclater les granules d'amidon (par exemple séchage et cuisson dans un tambour sécheur) ;
- oxydation par des oxydants forts conduisant à l'introduction de groupes carboxyle dans la molécule d'amidon et à la dépolymèrisation de la molécule d'amidon (par exemple en traitant une solution aqueuse d'amidon par l'hypochlorite de sodium) ;
- réticulation par des agents fonctionnels capables de réagir avec les groupes hydroxyle des molécules d'amidon qui vont ainsi être liées entre elles (par exemple avec des groupes glyceryl et/ou phosphate) ;
- estérification en milieu alcalin pour le greffage de groupes fonctionnels, notamment acétyl, hydroxyéthyl, hydroxypropyl, carboxyméthyl, octénylsuccinique.

Comme amidons modifiés utilisables selon l'invention, on peut citer par exemple les amidons estérifiés par l'anhydride octénylsuccinique et plus particulièrement le "Aluminium Starch octenyl succinate" tel que le produit vendu par la société NATIONAL STARCH sous le nom de DRY-FLO, l'amidon de maïs réticulé vendu sous le nom RESISTAMYL E2 par !a société AMYLUM ; l'amidon de pomme de terre estérifié par un groupe carboxyméthyl vendu sous le nom SUPRAMYL P 60 par la société AMYLUM, l'amidon de maïs estérifié par un groupe hydroxypropyle vendu sous le nom MERIGEL EF6 par la société AMYLUM ; l'amidon prégélatimisé, modifié par l'anhydride octénylsuccinique, puis par un motif hydrophobe, vendu sous le nom de NATROSORB HFB par la société NATIONAL STARCH ; l'amidon de maïs réticulé et acétylé vendu par la société CERESTAR sous le nom C* Flo 06205.

Selon un mode préféré de réalisation de l'invention, les amidons utilisés sont le DRY-FLO et le C* Flo 06205.

La phase huileuse constitue de 50 à 95 % et de préférence de 60 à 90 % en poids du poids total de la composition. Elle contient au moins une huile démaquillante choisie parmi les esters d'acide gras comportant au moins 12 atomes de carbone.

Ces esters sont de préférence obtenus à partir d'un alcool à chaîne droite ou ramifiée, comportant de 1 à 17 atomes de carbone et d'un acide gras à chaîne droite ou ramifiée, comportant au moins 12 atomes de carbone et de préférence de 14 à 22 atomes de carbone. Il s'agit de préférence de mono- ou de di-esters.

De manière préférée, l'huile démaquillante est choisie dans le groupe des esters qui ne comportent aucune insaturation et/ou aucun groupement éther ou hydroxyle. De façon encore plus avantageuse, l'huile démaquillante est un ester saturé qui ne renferme aucun groupement éther ni hydroxyle.

Ainsi, l'huile démaquillante de la composition conforme à l'invention peut être notamment choisie dans le groupe comprenant le palmitate d'éthyl-2 hexyle (ou palmitate d'octyle), le myristate d'éthyl-2 hexyle (ou myristate d'octyle), le palmitate d'isopropyle, le myristate d'isopropyle, l'adipate de di-isopropyle, l'hexanoate d'éthyl-2 hexyle, le laurate d'éthyle, le myristate de méthyle, l'octanoate d'octyldodécyle, le néopentanoate d'isodécyle, le myristate d'éthyle, le propionate de myristyle, l'éthyl-2 hexanoate d'éthyl-2 hexyle, l'octanoate d'éthyl-2 hexyle, le caprate/caprylate d'éthyl-2 hexyle, le palmitate de méthyle, le myristate de butyle, le myristate d'isobutyle, le palmitate d'éthyle, le laurate d'isohexyle, le laurate d'hexyle, l'isostéarate d'isopropyle, et leurs mélanges.

En plus de la ou des huiles démaquillantes, la phase huileuse peut contenir au moins une huile non démaquillante qui peut être choisie parmi les huiles minérales telles que les huiles de paraffine ou la vaseline, les huiles de silicone telles que les huiles de silicone volatile, les huiles d'origine végétale (par exemple l'huile d'amandes douces, l'huile d'amandes d'abricot), les huiles d'origine animale, les huiles de synthèse autres que les esters d'acide gras comportant au moins 12 atomes de carbone, et leurs mélanges.

La phase huileuse peut également contenir une ou plusieurs matières grasses telles que les acides gras, les alcools gras, les cires telles que les cires d'origine animale comme la cire d'abeille, les cires de carnauba ou de candellila, les cires minérales comme les cires microcristallines et les cires de synthèse comme les cires de polyéthylène ou de silicone.

Quand la phase huileuse n'est pas uniquement constituée d'huile(s) démaquillante(s), elle contient au moins 3 % en poids et de préférence au moins 10 % en poids d'huile(s) démaquillante(s) par rapport au poids total de la poudre.

La poudre de l'invention peut comprendre aussi un ou plusieurs additifs couramment utilisés dans le domaine cosmétique. Comme additifs, on peut citer par exemple les actifs cosmétiques, les matières colorantes y compris les pigments, les abrasifs, les agents antioxydants ou anti-radicaux libres, les charges, les parfums. Ces additifs peuvent représenter de 0 à 30 % du poids total de la poudre, de préférence de 0,5 à 15 % du poids total de la poudre.

Comme actifs, on peut citer par exemple les agents anti-acné, antimicrobiens, les agents de conditionnement de la peau, les vitamines, les acides gras essentiels, les agents kératolytiques, les enzymes, les agents hydratants comme les polyols et notamment la glycérine, et tout actif approprié dans une composition démaquillante.

De façon avantageuse, la poudre cosmétique de l'invention comporte un milieu. physiologiquement acceptable pour la peau, le cuir chevelu et/ou les muqueuses. Elle constitue notamment une poudre de démaquillage et/ou de nettoyage.

Un autre objet de l'invention concerne un procédé de fabrication d'une poudre cosmétique, comprenant au moins un amidon modifié et une phase huileuse comprenant au moins une huile démaquillante, le dit procédé comprenant (1) la préparation d'une dispersion huile-dans-eau par mélange d'une phase huileuse comprenant au moins une huile démaquillante choisie parmi les esters d'acide gras comportant au moins 12 atomes de carbone, dans une phase aqueuse comprenant au moins un amidon modifié, le rapport pondéral phase huileuse/amidon étant égal ou supérieur à 1 et la quantité d'huile(s) démaquillante(s) étant d'an moins 10% en poids par rapport au poids total de la poudre, et (2) la déshydratation de ladite dispersion pour obtenir ladite poudre.

Par dispersion, on entend toute dispersion ou émulsion huile-dans-eau, c'est-à-dire tout mélange d'une phase huileuse dans une phase aqueuse en présence ou non d'un émulsionnant.

La phase aqueuse de la dispersion huile-dans-l'eau représente de préférence au moins 30% en poids de la dispersion.

Le rapport pondéral phase huileuse/amidon est égal ou supérieur à 1 et va de préférence de 1 à 19 et mieux de 2 à 10.

Selon un mode particulier de réalisation du procédé, la dispersion utilisée est telle qu'elle a une teneur en matière sèche allant de 5 à 70 % en poids et de préférence allant de 10 à 60 % en poids. Une telle teneur en matière sèche permet d'avoir une viscosité de la dispersion telle qu'elle soit suffisamment fluide pour pouvoir être utilisée pour la suite des étapes.

La phase aqueuse peut être préparée à toute température (0 à 100 °C). On travaille de préférence à une température allant de 80° à 100° C.

De manière parallèle, on prépare la phase huileuse et on l'ajoute à la phase aqueuse qui est de préférence refroidie à une température inférieure à 80°, de préférence en une quantité telle qu'on a, dans la dispersion qui en résulte, une teneur en matière sèche allant de 5 à 70 % en poids. L'incorporation de la phase huileuse dans la phase aqueuse est réalisée tout en maintenant une agitation constante.

On obtient ainsi une dispersion huile-dans-eau, présentant un pH dépendant de sa composition, mais généralement compris entre 4 et 9.

De manière préférée, le procédé comprend une étape d'homogénéisation entre la préparation de la dispersion par mélange de la phase huileuse et de la phase aqueuse et avant la déshydratation de la dispersion obtenue. L'homogénéisation est avantageusement réalisée sous pression élevée, de manière à diminuer la taille moyenne des gouttelettes de la phase huileuse jusqu'à environ 350 nm, voire moins. L'homogénéisation est réalisée sous une pression en général allant de 300 à 600 bars, de préférence d'environ 600 bars (60 x 10⁶ Pa), pour obtenir des gouttelettes de phase huileuse ayant une taille moyenne (en nombre) de préférence inférieure à 350 nm, allant généralement de 80 à 300 nm.

La dispersion ainsi homogénéisée est alors déshydratée par tout procédé connu, et notamment par atomisation ou par lyophilisation. Selon un mode préféré de réalisation de l'invention, la déshydratation est effectuée par atomisation. Dans ce cas, la température de l'air chaud utilisé pour le séchage va de préférence d'environ 100°C à 220°C, et la température de sortie de la poudre va de préférence d'environ 30°C à 140°C. Le temps d'atomisation est très bref ; il est de préférence inférieur ou égal à 2 minutes.

La présente invention a aussi pour objet une poudre cosmétique susceptible d'être obtenue selon le procédé décrit ci-dessus.

La poudre obtenue peut être compactée pour gagner du volume et pour en faciliter le conditionnement, la conservation, le stockage et l'emploi. Avant le compactage éventuel de la poudre, celle-ci peut subir une étape de granulation supplémentaire ayant pour but d'homogénéiser la granulométrie de la poudre.

La poudre obtenue est très stable et se conserve plusieurs mois sans que l'on puisse observer de séparation de phases, d'évolution de la couleur, de reprise d'eau ou d'autres dégradations, microbiologiques par exemple.

En outre, les actifs sensibles à l'eau ou à l'oxydation, tels que des vitamines comme l'acide ascorbique (vitamine C), le thé vert ou les enzymes, restent très stables dans ce type de poudre.

La poudre de l'invention peut être utilisée directement telle quelle ; elle permet alors un bon nettoyage à sec d'une zone à démaquiller par application directe de la poudre et massage avec les doigts. Elle peut aussi être incorporée dans une composition aqueuse ou simplement dans de l'eau au moment de l'emploi ; on reconstitue alors une composition de démaquillage et/ou de nettoyage sous forme d'une émulsion H/E très riche en huiles, ne contenant ni tensioactifs ni conservateurs et donnant un très bon résultat de démaquillage.

La présente invention a donc aussi pour objet une composition de démaquillage et/ou de nettoyage comprenant une poudre telle que définie ci-dessus.

La présente invention a encore pour objet un procédé cosmétique de démaquillage et/ou de nettoyage de la peau, des muqueuses et/ou du cuir chevelu, comprenant l'application sur la peau, les muqueuses et/ou le cuir chevelu, d'une poudre telle que définie précédemment ou de la composition contenant la dite poudre.

La poudre selon l'invention ou une composition la contenant est particulièrement appropriée pour le démaquillage des compositions de maquillage sans transfert.

Aussi, l'invention a encore pour objet l'utilisation cosmétique de la poudre telle que définie ci-dessus ou de la composition contenant la dite poudre, pour le démaquillage d'une composition de maquillage longue tenue et/ou sans transfert.

L'invention est illustrée plus en détail à l'aide de l'exemple qui suit. Les pourcentages y sont donnés en poids.

### Exemple 1: Poudre de démaquillage de la peau

- Amidon modifié (DRY FLO) 22 %
- Glycérine 3 %
- Palmitate d'éthyl-2 hexyle 75 %

Pour obtenir cette poudre, on prépare un mélange contenant 8 % d'amidon modifié et 64,7 % d'eau et de glycérine, et on chauffe le mélange à 95°C, puis on le refroidit à 70 °C avant d'incorporer 27,3 % de palmitate d'éthyl-2 hexyle, tout en maintenant une agitation constante de manière à obtenir une dispersion huile-dans-eau. On refroidit la dispersion jusqu'à température ambiante tout en l'agitant et on l'homogénéise sous une pression d'environ 600 bars (60 x 10⁸ Pa), puis on la passe dans un appareil d'atomisation où l'air chaud est à 150°C et la température de sortie est de 120°C.

La poudre est utilisée telle quelle, non reconstituée, sur le visage. Après application directe de la poudre, on la masse avec les doigts, puis on passe un coton inhibé d'eau ou de tonique. La peau est parfaitement démaquillée et est fraîche, souple et satinée.

## Revendications

1. Poudre cosmétique de démaquillage et/ou de nettoyage, **caractérisée en ce qu**'elle comprend, par rapport au poids total de la composition, de 5 à 50 % en poids d'au moins un amidon modifié et de 50 à 95 % en poids d'une phase huileuse comprenant au moins une huile démaquillante choisie parmi les esters d'acide gras comportant au moins 12 atomes de carbone, le rapport pondéral phase huileuse/amidon étant égal ou supérieur à 1 et la quantité d'huile(s) démaquillante(s) étant d'au moins 10 % en poids par rapport au poids total de la poudre.

2. Poudre selon la revendication 1, **caractérisée en ce qu**'elle est exempte de protéine.

3. Poudre selon l'une quelconque des revendications précédentes, **caractérisée en ce que** l'amidon est modifié par au moins une réaction choisie parmi la prégélatinisation, l'oxydation, la réticulation et l'estérification.

4. Poudre selon l'une quelconque des revendications précédentes, **caractérisée en ce que** l'amidon modifié est l'amidon estérifié par l'anhydride octénylsuccinique.

5. Poudre selon l'une quelconque des revendications précédentes, **caractérisée en ce que** l'huile démaquillante est choisie parmi les esters obtenus à partir d'un alcool à chaîne droite ou ramifiée, comportant de 1 à 17 atomes de carbone et d'un acide gras à chaîne droite ou ramifiée, comportant de 14 à 22 atomes de carbone.

6. Poudre selon l'une quelconque des revendications 1 à 4, **caractérisée en ce que** l'huile démaquillante est choisie dans le groupe comprenant le palmitate d'éthyl-2 hexyle, le myristate d'éthyl-2 hexyle, le palmitate d'isopropyle, le myristate d'isopropyle, l'adipate de di-isopropyle, l'hexanoate d'éthyl-2 hexyle, le laurate d'éthyle, le myristate de méthyle, l'octanoate d'octyldodécyle, le néopentanoate d'isodécyle, le myristate d'éthyle, le propionate de myristyle, l'éthyl-2 hexanoate d'éthyl-2 hexyle, l'octanoate d'éthyl-2 hexyle, le caprate/caprylate d'éthyl-2 hexyle, le palmitate de méthyle, le myristate de butyle, le myristate d'isobutyle, le palmitate d'éthyle, le laurate d'isohexyle, le laurate d'hexyle, l'isostéarate d'isopropyle et leurs mélanges.

7. Poudre selon l'une quelconque des revendications précédentes, **caractérisée. en ce que** la phase huileuse contient en outre au moins une huile non démaquillante et/ou au moins une matière grasse choisie parmi les acides gras, les alcools gras et les cires.

8. Poudre selon l'une quelconque des revendications précédentes, **caractérisée en ce qu**'elle contient en outre, au moins un additif choisi parmi les actifs cosmétiques, les matières colorantes, les abrasifs, les agents antioxydants ou anti-radicaux libres, les parfums et les charges.

9. Procédé de fabrication d'une poudre cosmétique, comprenant au moins un amidon modifié et une phase huileuse comprenant au moins une huile démaquillante, comprenant (1) la préparation d'une dispersion huile-dans-eau par mélange d'une phase huileuse comprenant au moins une huile démaquillante choisie parmi les esters d'acide gras comportant au moins 12 atomes de carbone, dans une phase aqueuse comprenant au moins un amidon modifié, le rapport pondéral phase huileuse/amidon étant égal ou supérieur à 1 et la quantité d'huile(s) démaquillante(s) étant d'au moins 10 % en poids par rapport au poids total de la poudre, et (2) la déshydratation de ladite dispersion pour obtenir ladite poudre.

10. Procédé selon la revendication précédente, **caractérisé en ce que** le rapport pondéral phase huileuse/amidon est de 1 à 19.

11. Procédé selon la revendication 9 ou 10, **caractérisé en ce que** la phase aqueuse de la dispersion huile-dans-eau représente au moins 30 % du poids total de la dispersion.

12. Procédé selon l'une quelconque des revendications 9 à 11, **caractérisé en ce qu**'il comprend une étape d'homogénéisation entre la préparation de la dispersion et avant la déshydratation de la dite dispersion.

13. Procédé selon l'une quelconque des revendications 9 à 12, **caractérisé en ce que** la déshydratation est effectuée par atomisation.

14. Procédé selon la revendication précédente, **caractérisé en ce que** la déshydratation est réalisée dans un appareil d'atomisation où la température de l'air chaud utilisé pour le séchage va de 100°C à 220°C, et la température de sortie de la poudre va de 30°C à 140°C.

15. Poudre cosmétique susceptible d'être obtenue par le procédé selon l'une quelconque des revendications 9 à 14.

16. Composition de démaquillage et/ou de nettoyage comprenant une poudre selon l'une quelconque des revendications 1 à 8 ou 15.

17. Procédé cosmétique de démaquillage et/ou de nettoyage de la peau, des muqueuses et/ou du cuir chevelu, comprenant l'application sur la peau, les. muqueuses et/ou le cuir chevelu, d'une poudre selon l'une quelconque des revendications 1 à 8 et 15 ou d'une composition selon la revendication 16.

18. Utilisation cosmétique de la poudre selon l'une quelconque des revendications 1 à 8 et 15 ou d'une composition selon la revendication 16, pour le démaquillage d'une composition de maquillage longue tenue et/ou sans transfert.

## Claims

1. Make-up-removing and/or cleansing cosmetic powder, **characterized in that** it comprises, relative to the total weight of the composition, from 5 to 50% by weight of at least one modified starch and from 50 to 95% by weight of an oily phase comprising at least one make-up-removing oil chosen from fatty acid esters comprising at least 12 carbon atoms, the oily phase/starch weight ratio being greater than or equal to 1 and the amount of make-up-removing oil(s) being at least 10% by weight relative to the total weight of the powder.

2. Powder according to Claim 1, **characterized in that** it is free of protein.

3. Powder according to either of the preceding claims, **characterized in that** the starch is modified by at least one reaction chosen from pregelatinization, oxidation, crosslinking and esterification.

4. Powder according to any one of the preceding claims, **characterized in that** the modified starch is starch esterified with octenylsuccinic anhydride.

5. Powder according to any one of the preceding claims, **characterized in that** the make-up-removing oil is chosen from esters obtained from a straight-chain or branched-chain alcohol comprising from 1 to 17 carbon atoms and a straight-chain or branched-chain fatty acid comprising from 14 to 22 carbon atoms.

6. Powder according to any one of Claims 1 to 4, **characterized in that** the make-up-removing oil is chosen from the group comprising 2-ethylhexyl palmitate, 2-ethylhexyl myristate, isopropyl palmitate, isopropyl myristate, diisopropyl adipate, 2-ethylhexyl hexanoate, ethyl laurate, methyl myristate, octyldodecyl octanoate, isodecyl neopentanoate, ethyl myristate, myristyl propionate, 2-ethylhexyl 2-ethylhexanoate, 2-ethylhexyl octanoate, 2-ethylhexyl caprate/caprylate, methyl palmitate, butyl myristate, isobutyl myristate, ethyl palmitate, isohexyl laurate, hexyl laurate and isopropyl isostearate, and mixtures thereof.

7. Powder according to any one of the preceding claims, **characterized in that** the oily phase also contains at least one non-make-up-removing oil and/or at least one fatty substance chosen from fatty acids, fatty alcohols and waxes.

8. Powder according to any one of the preceding claims, **characterized in that** it also contains at least one additive chosen from cosmetic active agents, dyestuffs, abrasive agents, antioxidants, free-radical scavengers, fragrances and fillers.

9. Process for manufacturing a cosmetic powder, comprising at least one modified starch and an oily phase comprising at least one make-up-removing oil, the said process comprising (1) the preparation of an oil-in-water dispersion by mixing an oily phase comprising at least one make-up-removing oil chosen from fatty acid esters comprising at least 12 carbon atoms, into an aqueous phase comprising at least one modified starch, the oily phase/starch weight ratio being greater than or equal to 1 and the amount of make-up-removing oil(s) being at least 10% by weight relative to the total weight of the powder, and (2) the dehydration of the said dispersion in order to obtain the said powder.

10. Process according to the preceding claim, **characterized in that** the oily phase/starch weight ratio is from 1 to 19.

11. Process according to Claim 9 or 10, **characterized in that** the aqueous phase of the oil-in-water dispersion represents at least 30% of the total weight of the dispersion.

12. Process according to any one of Claims 9 to 11, **characterized in that** it comprises a homogenization step between the preparation of the dispersion and before the dehydration of the said dispersion.

13. Process according to any one of Claims 9 to 12, **characterized in that** the dehydration is carried out by spraying.

14. Process according to the preceding claim, **characterized in that** the dehydration is carried out in a spraying machine in which the temperature of the hot air used for the drying ranges from 100°C to 220°C and the outlet temperature of the powder ranges from 30°C to 140°C.

15. Cosmetic powder which can be obtained by the process according to any one of Claims 9 to 14.

16. Make-up-removing and/or cleansing composition comprising a powder according to any one of Claims 1 to 8 or 15.

17. Cosmetic process for removing make-up from and/or for cleansing the skin, mucous membranes and/or the scalp, comprising the application of a powder according to any one of Claims 1 to 8 and 15 or of a composition according to Claim 16 to the skin, mucous membranes and/or the scalp.

18. Cosmetic use of the powder according to any one of Claims 1 to 8 and 15 or of a composition according to Claim 16, for removing a long-lasting and/or transfer-resistant make-up composition.

## Patentansprüche

1. Kosmetisches Pulver zum Abschminken und/oder zum Reinigen, **dadurch gekennzeichnet**, daß es, bezogen auf das Gesamtgewicht der Zusammensetzung, 2 bis 50 Gew.-% mindestens einer modifizierten Stärke und 50 bis 95 Gew.-% Ölphase enthält, die mindestens ein Öl zum Abschminken enthält, das unter den Estern einer Fettsäure mit mindestens 12 Kohlenstoffatomen ausgewählt ist, wobei das Gewichtsverhältnis Ölphase/Stärke mindestens 1 ist und die Menge des Öls oder der Öle zum Abschminken mindestens 10 Gew.-%, bezogen auf das Gesamtgewicht der Zusammensetzung, ausmacht.

2. Pulver nach Anspruch 1, **dadurch gekennzeichnet**, daß es keine Proteine enthält.

3. Pulver nach einem der vorhergehenden Ansprüche, **dadurch gekennzeichnet**, daß die Stärke durch mindestens eine Reaktion modifiziert wurde, die unter Verkleisterung, Oxidation, Vernetzung und Veresterung ausgewählt ist.

4. Pulver nach einem der vorhergehenden Ansprüche, **dadurch gekennzeichnet**, daß es sich bei der modifizierten Stärke um mit Octenylbernsteinsäureanhydrid veresterte Stärke handelt.

5. Pulver nach einem der vorhergehenden Ansprüche, **dadurch gekennzeichnet**, daß das Öl zum Abschminken unter den Estern ausgewählt ist, die ausgehend von einem geradkettigen oder verzweigten Alkohol mit 1 bis 17 Kohlenstoffatomen und einer geradkettigen oder verzweigten Fettsäure mit 14 bis 22 Kohlenstoffatomen hergestellt ist.

6. Pulver nach einem der Ansprüche 1 bis 4, **dadurch gekennzeichnet**, daß das Öl zum Abschminken unter 2-Ethylhexylpalmitat, 2-Ethylhexylmyristat, Isopropylpalmitat, Isopropylmyristat, Diisopropyladipat, 2-Ethylhexylhexanoat, Ethyllaurat, Methylmyristat, Octyldodecyloctanoat, Isodecylneopentanoat, Ethylmyristat, Myristylpropionat, 2-Ethylhexyl-2-ethylhexanoat, 2-Ethylhexyloctanoat, 2-Ethylhexylcaprat/caprylat, Methylpalmitat, Butylmyristat, Isobutylmyristat, Ethylpalmitat, Isohexyllaurat, Hexyllaurat, Isopropylisostearat und deren Gemischen ausgewählt ist.

7. Pulver nach einem der vorhergehenden Ansprüche, **dadurch gekennzeichnet**, daß die Ölphase mindestens ein Öl, das nicht zum Abschminken dient, und/oder mindestens eine Fettsubstanz enthält, die unter den Fettsäuren, Fettalkoholen und Wachsen ausgewählt ist.

8. Pulver nach einem der vorhergehenden Ansprüche, **dadurch gekennzeichnet**, daß es ferner mindestens einen Zusatzstoff enthält, der unter den kosmetischen Wirkstoffen, Färbemitteln, abrasiven Mitteln, Antioxidantien, Mitteln gegen freie Radikale, Parfums und Füllstoffen ausgewählt ist.

9. Verfahren zur Herstellung eines kosmetischen Pulvers, das mindestens eine modifizierte Stärke und eine Ölphase mit mindestens einem Öl zum Abschminken enthält, das (1) die Herstellung einer Öl-in-Wasser-Dispersion durch Mischen einer Ölphase mit mindestens einem Öl zum Abschminken, das unter den Estern einer Fettsäure mit mindestens 12 Kohlenstoffatomen ausgewählt ist, und einer wäßrigen Phase, die mindestens eine modifizierte Stärke enthält, wobei das Gewichtsverhältnis Ölphase/Stärke mindestens 1 beträgt und die Menge des Öls oder der Öle zum Abschminken mindestens 10 Gew.-%, bezogen auf das Gesamtgewicht des Pulvers, ausmacht, und (2) die Dehydratisierung der Dispersion zur Herstellung eines Pulvers umfaßt.

10. Verfahren nach dem vorhergehenden Anspruch, **dadurch gekennzeichnet**, daß das Gewichtsverhältnis Ölphase/Stärke im Bereich von 1 bis 19 liegt.

11. Verfahren nach Anspruch 9 oder 10, **dadurch gekennzeichnet**, daß die wäßrige Phase der Öl-in-Wasser-Dispersion mindestens 30 % des Gesamtgewichts der Dispersion ausmacht.

12. Verfahren nach einem der Ansprüche 9 bis 11, **dadurch gekennzeichnet**, daß es nach der Herstellung der Dispersion und vor der Dehydratisierung der Dispersion einen Homogenisierungsschritt umfaßt.

13. Verfahren nach einem der Ansprüche 9 bis 12, **dadurch gekennzeichnet**, daß die Dehydratisierung durch Zerstäuben durchgeführt wird.

14. Verfahren nach dem vorhergehenden Anspruch, **dadurch gekennzeichnet**, daß die Dehydratisierung in einer Vorrichtung zum Zerstäuben durchgeführt wird, in der die Temperatur der zum Trocknen verwendeten heißen Luft im Bereich von 100 bis 220 °C und die Temperatur des Pulvers am Auslaß im Bereich von 30 bis 140 °C liegt.

15. Kosmetisches Pulver, das nach dem Verfahren nach einem der Ansprüche 9 bis 14 erhältlich ist.

16. Zusammensetzung zum Abschminken und/oder zur Reinigung, die ein Pulver nach einern der Ansprüche 1 bis 8 oder 15 enthält.

17. Kosmetisches Verfahren zum Abschminken und/oder Reinigen der Haut, der Schleimhäute und/oder der Kopfhaut, das das Aufbringen eines Pulvers nach einem der Ansprüche 1 bis 8 und 15 oder einer Zusammensetzung nach Anspruch 16 auf die Haut, die Schleimhäute und/oder die Kopfhaut umfaßt.

18. Kosmetische Verwendung des Pulvers nach einem der Ansprüche 1 bis 8 und 15 oder einer Zusammensetzung nach Anspruch 18 zum Abschminken einer Zusammensetzung zum Schminken mit langer Haltbarkeit und/oder ohne Transfer.
